Europäisches Patentamt

**European Patent Office**

Office européen des brevets

(19)

(11) Veröffentlichungsnummer: **0 009 159**
B1

(12) EUROPÄISCHE PATENTSCHRIFT

(45) Veröffentlichungstag der Patentschrift:
23.09.81

(21) Anmeldenummer: 79103247.7

(22) Anmeldetag: 03.09.79

(51) Int. Cl.³: **C 07 C 69/145,** C 07 C 67/293,
C 07 C 67/29

(54) Verfahren zur Herstellung von Carbonsäureestern des Beta-Formyl-Crotylalkohols mittels einer Allylumlagerung.

(30) Priorität: 15.09.78 DE 2840125

(43) Veröffentlichungstag der Anmeldung:
02.04.80 Patentblatt 80/7

(45) Bekanntmachung des Hinweises auf die Patenterteilung:
23.09.81 Patentblatt 81/38

(84) Benannte Vertragsstaaten:
BE CH DE FR GB NL

(56) Entgegenhaltungen:
DE-A-1 297 597
FR-A-1 576 842

(73) Patentinhaber: **BASF Aktiengesellschaft,
Carl-Bosch-Strasse 38, D-6700 Ludwigshafen (DE)**

(72) Erfinder: **Laas, Harald, Dr., Dipl.-Chem.,
Woehlerstrasse 14, D-6701 Maxdorf 2 (DE)**
Erfinder: **Nissen, Axel, Dr., Dipl.-Chem.,
Panoramastrasse 51, D-6906 Leimen (DE)**
Erfinder: **Meissner, Bernd, Dr., Dipl.-Chem.,
Dammweg 15, D-6900 Heidelberg (DE)**

Verfahren zur Herstellung von Carbonsäureestern des $\beta$-Formyl-Crotyl-
alkohols mittels einer Allylumlagerung

Die Erfindung betrifft eine Verbesserung eines Verfahrens zur Herstellung von Carbonsäureestern des $\beta$-Formyl-crotylalkohols durch Umlagerung der entsprechenden Carbonsäureester des 2-Formyl-2-hydroxy-3-butens bzw. eines Acetals oder Acylats davon in Gegenwart von Kupfer oder von Kupferverbindungen und gegebenenfalls anschließende hydrolytische Spaltung der Acetal- oder Acylatgruppierung und insbesondere eine Verbesserung der Herstellung von 4,4-Dimethoxy-3-methyl-crotylacetat (3-Dimethoxy-methyl-crotylacetat) aus 1,1-Dimethoxy-2-methyl-2-acetoxy-1-buten.

Die Carbonsäureester des $\beta$-Formyl-crotylalkohols haben bekanntlich große technische Bedeutung, da sie u. a. als Ausgangsmaterial für eine technische Synthese von Vitamin A und dessen Abkömmlingen Verwendung finden. Es wurden daher große Anstrengungen unternommen, um ein technisch vorteilhaftes Verfahren zu ihrer Herstellung zu finden. Das bislang beste Verfahren ist wohl das aus der deutschen Patentschrift 1 297 597 bekannte Verfahren, gemäß dem Carbonsäureester des $\beta$-Formyl-crotylalkohols durch Erhitzen der entsprechenden Carbonsäureester des 2-Formyl-2-hydroxy-3-butens bzw. eines Acetals oder Acylat, davon in Gegenwart von Kupfer oder von Kupferverbindungen und gegebenenfalls anschließende hydrolytische Spaltung der Acetal- oder Acylat-Gruppierung erhalten werden. Die Allylumlagerung erfolgt hierbei mit Ausbeuten von bis zu 72,8%, die anschließende hydrolytische Spaltung praktisch quantitativ.

Die gemäß diesem Verfahren erzielten Ausbeuten befriedigten technisch noch nicht und so war es die Aufgabe der Erfindung, das beschriebene Verfahren bezüglich der erreichbaren Ausbeuten an $\beta$-Formyl-crotylacetat noch zu verbessern.

Es wurde nun überraschenderweise gefunden, daß man die Carbonsäureester von $\beta$-Formylcrotylalkohol durch die beschriebene Allylumlagerung in Gegenwart von Kupferverbindungen mit Ausbeuten von mehr als 80% und in sehr guten Raum-Zeit-Ausbeuten erhalten kann, wenn man einerseits die Umlagerung in Gegenwart von Kupfer(I)-chlorid als Katalysator vornimmt und andererseits dafür sorgt, daß die sich während der Umlagerung durch Abspaltungsreaktionen als Nebenprodukte bildenden niedrigsiedenden Substanzen wie beispielsweise Methanol, Methylacetat, Essigsäure und Wasser kontinuierlich aus dem Reaktionsgemisch entfernt werden.

Gegenstand der Erfindung ist daher ein verbessertes Verfahren zur Herstellung von Carbonsäureestern des -Formyl-crotylalkohols durch Umlagerung der entsprechenden Carbonsäureester des 2-Formyl-2-hydroxy-3-butens bzw. eines Acetals oder Acylats, davon in Gegenwart von Kupfer oder Kupferverbindungen bei Temperaturen von 50 bis 250°C, vorzugsweise 110 bis 180°C und gegebenenfalls anschließende hydrolytische Spaltung der Acetal- oder Acylatgruppierung, das dadurch gekennzeichnet ist, daß man

a)    die Umlagerung in Gegenwart von Kupfer(I)-chlorid als Katalysator vornimmt und
b)    die sich während der Umlagerung bildenden niedersiedenden Nebenprodukte in an sich bekannter Weise aus dem Reaktionsgemisch entfernt.

Das Entfernen der sich während der Umlagerung bildenden niedersiedenden Nebenprodukte kann auf destillative Weise oder ebenso vorteilhaft durch Strippen mit einem das Reaktionsgemisch durchströmenden inerten Gases erfolgen.

Die Ausgangsstoffe können in üblicher Weise durch Acylierung von 2-Formyl-2-hydroxy-buten-(3) bzw. von dessen Acetalen oder Acylaten mit Säurehalogeniden oder Säureanhydriden geeigneter Carbonsäuren hergestellt werden. Man kann die Ausgangsstoffe auch durch Acylierung von 2-Formyl-2-hydroxy-buten-(3) bzw. dessen Acetalen oder Acylaten und nachfolgende partielle Hydrierung der Ester erhalten.

Der Estergruppe in den Ausgangsstoffen können beispielsweise aliphatische, cycloaliphatische, araliphatische oder aromatische Carbonsäuren zugrunde liegen, die im allgemeinen 1 bis 18 Kohlenstoffatome aufweisen und auch noch inerte Substituenten wie Halogenatome tragen können. Bevorzugt verwendet man solche Ausgangsstoffe, deren Estergruppe sich von einfachen aliphatischen Carbonsäuren mit 1 bis 4 Kohlenstoffatomen ableitet. Beispiele für Carbonsäuren, die der Estergruppe zugrunde liegen können, sind Hexahydrobenzoesäure, Propionsäure, Benzoesäure oder Palmitinsäure, insbesondere aber Essigsäure.

Als Ausgangsstoffe können Carbonsäureester des 2-Formyl-2-hydroxy-butens-(3) selbst oder eines Acetals oder Acylats davon verwendet werden, wobei die Acetale vorzugsweise eingesetzt werden.

Von den Acetalen verwendet man bevorzugt solche, die sich von einfachen aliphatischen Alkoholen mit 1 bis 4 Kohlenstoffatomen ableiten, wie Methylalkohol, Äthylalkohol oder Isopropylalkohol. Als Acylate werden diejenigen bevorzugt, denen einfache aliphatische Carbonsäuren mit 1 bis 4 Kohlenstoffatomen zugrunde liegen.

Beispiele der bei dem Verfahren dieser Erfindung zu verwendenden Ausgangsstoffe sind 1,1-Dimethoxy-, 1,1-Diäthoxy-, 1,1-Di-n-butoxy-2-methyl-2-acetoxy-3-buten, 1,1-Dimethoxy-2-methyl-2-benzoyloxy-3-buten, 2-(Phenylacetoxy)-2-formyl-3-buten oder 1,1-Diacetoxy-2-methyl-(chloracetoxy)-3-buten. Aus praktischen Gründen werden 1,1-Dimethoxy- und 1,1-Diäthoxy-2-methyl-2-acetoxy-3-buten bevorzugt verwendet.

Als Katalysator für die Umlagerung wird Kupfer(I)-chlorid verwendet. Das Kupfer(I)-chlorid kann als solches oder auf einem inerten Trägermaterial eingesetzt werden. Man verwendet den Katalysator im allgemeinen in Mengen von 0,005 bis 5 Gewichtsprozent, zweckmäßig 0,05 bis 0,5 Gewichtsprozent, berechnet als Kupfer, bezogen auf den Ausgangsstoff.

Es war überraschend, daß die Verwendung von Kupfer(I)-chlorid in Verbindung mit dem Abtrennen des Niedersieder aus dem Reaktionsgemisch im Maße von deren Bildung eine Ausbeutesteigerung von etwa 10% mit sich bringt. Bei Verwendung von anderen Kupferverbindungen, wie z. B. von Kupferoxid, Kupferacetat, Kupferjodid, Kupferbromid oder Gemischen von Kupferverbindungen führt dagegen das Abtrennen der Niedersieder aus dem Reaktionsgemisch während der Umlagerung nur zu unwesentlichen Ausbeuteverbesserungen (siehe Vergleichsbeispiele).

Für die Umlagerung wählt man im allgemeinen Temperaturen zwischen 50 und 250°C, vorzugsweise zwischen 110 und 180°C. Bei tiefen Temperaturen verläuft die Reaktion sehr langsam. Bei Temperaturen oberhalb von 180°C besteht bereits die Gefahr der Bildung von Zersetzungsprodukten.

Zur Durchführung des erfindungsgemäßen Verfahrens erhitzt man die Ausgangsverbindung im allgemeinen in einem Reaktionsgefäß, das mit einem Rührer, einem Innenthermometer und einem abfallenden Kühler versehen ist, auf die Reaktionstemperatur. Das Abtrennen der sich bei Umlagerung bildenden niedrigsiedenden Nebenprodukte (Niedersieder) in situ kann durch kontinuierliches Abdestillieren aus dem Reaktionsgemisch sowohl bei Atmosphärendruck als auch bei vermindertem Druck, zweckmäßig bei einem Druck von 100 bis 500, vorzugsweise 200 bis 350 mbar erfolgen.

Die technische Durchführung der Abtrennung der niedrigsiedenden Komponenten ist aufgrund der hohen Siedepunktsunterschiede relativ einfach. Sowohl im Labor als auch im technischen Maßstab wird lediglich durch die Gestaltung des mit dem Reaktionsgefäß verbundenen Kühlers dafür gesorgt, daß die Niedersieder abdestillieren können, jedoch der Austrag an Wertprodukten unter 0,5% bleibt.

Das Abtrennen der Niedersieder durch Strippen mit einem das Reaktionsgemisch durchströmenden inerten Gases erfolgt zweckmäßig mit Stickstoff oder Argon; möglich, ist jedoch auch die Verwendung von Gasen wie $CO_2$, Methan, Wasserstoff oder Methylchlorid.

Um einen günstigen Strippeffekt zu erzielen, sind Gasmengen von etwa 20–300 Liter, vorzugsweise 100 bis 200 Liter Gas pro Kg eingesetzte Verbindung und pro Stunde erforderlich.

Verwendet man als Ausgangsstoffe Carbonsäureester des 2-Formyl-2-hydroxy-butens-(3), so erhält man nach der Umlagerung sogleich Carbonsäureester des β-Formyl-crotylalkohols. Setzt man dagegen als Ausgangsstoffe die zugehörigen Acetale oder Acylate ein, so erhält man als Umlagerungsprodukte Acetale bzw. Acylate von Carbonsäureestern des β-Formyl-crotylalkohols, die anschließend durch Hydrolyse in üblicher Weise quantitativ in die Endprodukte übergeführt werden. Man kann dazu die umgelagerten Acetale oder Acylate isolieren und anschließend in einer zweiten Operation hydrolysieren. Es kann jedoch auch vorteilhaft sein, die umgelagerten Acetale oder Acylate ohne Isolierung im selben Reaktionsgefäß zu den Endprodukten umzusetzen.

Die Umlagerungsreaktion nimmt je nach Temperatur im allgemeinen 2 bis 10, vorzugsweise 2 bis 6 Stunden in Anspruch. Die Hydrolysedauer der umgelagerten Acetale oder Acylate beträgt je nach Temperatur und pH-Wert des Reaktionsgemisches im allgemeinen 0,5 bis 2 Stunden.

Mit Hilfe des erfindungsgemäßen Verfahrens können die als Ausgangsmaterial für eine technische Synthese von Vitamin A und dessen Abkömmlinge begehrten Carbonsäureester des β-Formyl-crotylalkohols mit wesentlich besseren Ausbeuten als nach den bekannten Verfahren erhalten werden.

Die in den folgenden Beispielen angegebenen Teile sind Gewichtsteile.

Der Endpunkt der Umlagerungsreaktion wurde jeweils durch gaschromatographische Untersuchung kleiner Proben bestimmt.

### Beispiel 1

#### A) Erfindungsgemäße Umsetzung

In einem Reaktionsgefäß, das mit einem Rührer, einem Innenthermometer und einer Destillierbrücke versehen war, wurden 1800 Teile 1,1-Dimethoxy-2-methyl-2-acetoxy-3-buten zusammen mit 1 Teil Kupfer(I)-chlorid unter Rühren auf Temperaturen von 155 bis 160°C erhitzt, wobei der Grad der Umlagerung durch gaschromatographische Untersuchung kleiner Proben bestimmt wurde. Während der gesamten Reaktionszeit von 6 Stunden destillierten die gebildeten Niedersieder kontinuierlich ab. Die anschließende destillative Aufarbeitung des Reaktionsgemisches ergab 1444 Teile 4,4-Dimethoxy-3-methyl-crotylacetat. Das entspricht einer Ausbeute von 80,2% der Theorie.

#### B) Vergleichsbeispiele

a) Umsetzung in Gegenwart von Cu(II)-acetat, CuBr bzw. CuJ unter Rückflußkühlung

In einem Reaktionsgefäß, das mit einem Rührer, einem Innenthermometer und einem Rückflußkühler versehen war, wurden 1800 Teile 1,1-Dimethoxy-2-methyl-2-acetoxy-3-buten zusammen mit 3,6 Teilen Kupfer(II)-acetat unter Rühren auf 165°C erhitzt.

Während der Reaktionszeit von 12 Stunden, fiel die Reaktionstemperatur durch die unter Rückfluß siedenden Niedersieder langsam bis auf 125°C ab. Die anschließende destillative Aufarbeitung des Reaktionsgemisches ergab 1152 Teile 4,4-Dimethoxy-3-methyl-crotylacetat. Das entspricht einer Ausbeute von 64% der Theorie.

Bei der analogen Umsetzung mit CuBr bzw. CuJ wurden folgende Ergebnisse erzielt:

| Katalysator | Umsatz | Ausbeute |
|---|---|---|
| | [%] | [%] |
| CuBr | 98,7 | 67,8 |
| CuJ | 44,7 | 33,9 |

b) Umsetzung in Gegenwart von Cu(II)-acetat, CuBr, CuJ bzw. CuO unter kontinuierlichem Abdestillieren der Niedersieder

In dem unter A) beschriebenen Reaktionsgefäß wurden 1800 Teile 1,1-Dimethoxy-2-methyl-2-acetoxy-3-buten zusammen mit 3,6 Teilen Kupfer(II)-acetat unter Rühren 12 Stunden lang auf eine Temperatur von 152°C erhitzt. Während der gesamten Reaktionszeit destillierten die gebildeten Niedersieder kontinuierlich ab. Die anschließende destillative Aufarbeitung des Reaktionsgemisches ergab 1278 Teile 4,4-Dimethoxy-3-methyl-crotylacetat. Das entspricht einer Ausbeute von 71% der Theorie.

Bei der analogen Umsetzung mit CuBr, CuJ oder CuO wurden folgende Ergenisse erzielt:

| Katalysator | Umsatz | Ausbeute |
|---|---|---|
| | [%] | [%] |
| CuBr | 97,8 | 73,1 |
| CuJ | 46,9 | 36,2 |
| CuO | 98,6 | 74,1 |

Beispiel 2

A) Erfindungsgemäße Umsetzung

In einem Reaktionsgefäß, das mit einem Rührer, einem Innenthermometer und einer Destillierbrücke versehen war, wurden 564 Teile 1,1-Dimethoxy-2-methyl-2-acetoxy-3-buten zusammen mit 0,3 Teilen Kupfer(I)-chlorid unter Rühren bei einem Druck von 0,2 bar auf 147°C erhitzt. Während der 7,5 Stunden Reaktionszeit destillierten die gebildeten Niedersieder kontinuierlich ab und die Reaktionstemperatur stieg auf 157°C an. Die anschließende destillative Aufarbeitung des Reaktionsgemisches ergab 459 Teile 4,4-Dimethoxy-3-methyl-crotylacetat. Das entspricht einer Ausbeute von 81,4% der Theorie.

B) Vergleichsbeispiel

Umsetzung in Gegenwart von Kupfer(I)-chlorid ohne Entfernen der Niedersider

In einem Reaktionsgefäß, das mit einem Rührer, einem Innenthermometer und einem Rückflußkühler versehen war, wurden 564 Teile 1,1-Dimethoxy-2-methyl-2-acetoxy-3-buten zusammen mit 0,3 Teilen Kupfer(I)-chlorid unter Rühren auf 160°C erhitzt. Während der 4 Stunden Reaktionszeit fiel die Reaktionstemperatur durch den einsetzenden Rückfluß der entstehenden Niedersieder allmählich auf 146°C ab. Die anschließende destillative Aufarbeitung des Reaktionsgemisches ergab 422 Teile 4,4-Dimethoxy-3-methyl-crotylacetat. Das entspricht einer Ausbeute von 74,8% der Theorie.

Beispiel 3

Umlagerung

In einem Reaktionsgefäß, das mit einem Rührer, einem Innenthermometer und einer Destillationsbrücke versehen war, wurden 564 g 1,1-Dimethoxy-2-methyl-2-acetoxy-3-buten zusammen mit 0,3 g Kupfer(I)-chlorid unter Rühren auf 160°C erhitzt. Während der gesamten Reaktionszeit von 5 Stunden wurde kontinuierlich ein Stickstoffstrom (90 Liter/Stunde) durch das Reaktionsgefäß geleitet.

Die anschließende destillative Aufarbeitung des Reaktionsgemisches ergab 462 Teile 4,4-Dimethoxy-3-methyl-crotylacetat. Das entspricht einer Ausbeute von 81,9% der Theorie.

Hydrolyse

In einem zweistufigen Umlaufreaktor wurden 80 Teile 1,1-Dimethoxy-2-methyl-2-acetoxy-3-buten pro Stunde mit 70 Teilen Wasser unter Rückflußkühlung auf 80°C erhitzt. Die mittlere Verweilzeit betrug 30 Minuten. Nach destillativem Abtrennen des Wasser-Methanol-Gemisches erhielt man das $\beta$-Formyl-crotylacetat in praktisch quantitativer Ausbeute.

Patentansprüche

1. Verbessertes Verfahren zur Herstellung von Carbonsäureestern des $\beta$-Formyl-crotylalkohols durch Umlagerung der entsprechenden Carbonsäureester des 2-Formyl-2-hydroxy-3-butens bzw. eines Acetals oder Acylates davon in Gegenwart von Kupfer oder Kupferverbindungen bei Temperaturen von 50 bis 250°C und gegebenenfalls anschließende hydrolytische Spaltung der Acetal- oder Acylatgruppierung, dadurch gekennzeichnet, daß man

a)  die Umlagerung in Gegenwart von Kupfer-(I)-chlorid als Katalysator vornimmt und
b)  die sich während der Umlagerung bildenden niedersiedenden Nebenprodukte in an sich bekannter Weise kontinuierlich aus dem Reaktionsgemisch entfernt.

2. Verfahren gemäß Anspruch 1, dadurch gekennzeichnet, daß man die sich während der Umlagerung bildenden niedersiedenden Nebenprodukte auf destillative Weise kontinuierlich aus dem Reaktionsgemisch entfernt.

3. Verfahren gemäß Anspruch 1, dadurch gekennzeichnet, daß man die sich während der Umlagerung bildenden niedersiedenden Nebenprodukte durch Strippen mit einem das Reaktionsgemisch durchströmenden inerten Gas kontinuierlich aus dem Reaktionsgemisch entfernt.

## Claims

1. An improved process for the preparation of a carboxylic acid ester of $\beta$-formyl-crotyl alcohol by rearrangement of the corresponding carboxylic acid ester of 2-formyl-2-hydroxy-but-3-ene or of an acetal or acylate thereof in the presence of copper or a copper compound at from 50 to 250°C followed, where relevant, by hydrolytic cleavage of the acetal or acylate group, characterized in that

a)  the rearrangement is carried out in the presence of copper (I) chloride as the catalyst, and
b)  the low-boiling by-products formed during the rearrangement are continuously removed from the reaction mixture in a conventional manner.

2. A process as claimed in claim 1, characterized in that the low-boiling by-products formed during the rearrangement are continuously removed from the reaction mixture by distillation.

3. A process as claimed in claim 1, characterized in that the low-boiling by-products formed during the rearrangement are continuously removed from the reaction mixture by stripping with an inert gas flowing through the reaction mixture.

## Revendications

1. Procédé amélioré pour la préparation d'esters carboxyliques de l'alcool $\beta$-formyl-crotylique par transposition des esters carboxyliques correspondants du 2-formyl-2-hydroxy-3-butène ou d'un acétal ou acylate de celui-ci, en présence de cuivre ou de composés du cuivre, à des températures de 50 à 250°C, suivie éventuellement d'une dissociation hydrolytique du groupement acétal ou acylate, caractérisé en ce que:

a)  on procède à la transposition en présence de chlorure cuivreux en tant que catalyseur et
b)  de façon connue en soi, on élimine en continu du mélange réactionnel les produits secondaires à bas point d'ébullition qui se forment pendant la transposition.

2. Procédé selon la revendication 1, caractérisé en ce qu'on élimine en continu du mélange réactionnel, par voie de distillation, les produits secondaires à bas point d'ébullition qui se forment pendant la transposition.

3. Procédé selon la revendication 1, caractérisé en ce qu'on élimine en continu du mélange réactionnel les produits secondaires à bas point d'ébullition qui se forment pendant la transposition, en procédant par stripping avec un gaz inerte que l'on fait passer rapidement à travers le mélange réactionnel.